# EUROPEAN PATENT SPECIFICATION

(11) **EP 2 258 345 B1**
(45) Date of publication and mention of the grant of the patent: **21.09.2016**
(21) Application number: 10181264.2
(22) Date of filing: 23.08.2004
(51) Int. Cl.: A61K 9/14, A61K 9/20, A61K 31/425

(54) **Solid pharmaceutical dosage form comprising an HIV protease inhibitor solid dispersion**
Feste pharmazeutische Darreichungsform enthaltend eine feste Dispersion mit einem HIV Protease Inhibitor
Forme posologique pharmaceutique solide comprénant une dispersion solide d'inhibiteur de protease HIV

(30) Priority: 28.08.2003 US 650178
(43) Date of publication of application: 08.12.2010
(62) Divisional of application: 04816820.7
(73) Proprietor: AbbVie Inc., North Chicago, IL 60064 (US)
(72) Inventor: Rosenberg, Jörg, 67158, Ellerstadt (DE); Reinhold, Ulrich, 52076 Aachen (DE); Liepold, Bernd, 69221 Dossenheim (DE); Berndl, Gunther, 67098 Bad Dürkheim-Ungstein (DE); Breitenbach, Jörg, 68199, Mannheim (DE); Alani, Laman, Lansdale, PA 19446 (US); Ghosh, Soumojeet, Gurnee, IL 60031 (US)
(74) Representative: Adams, Harvey Vaughan John

(56) References cited:
- WO-A-01/34119
- WO-A-2004/032903
- US-A- 6 027 747

## Description

The present disclosure is directed to a solid pharmaceutical dosage form comprising at least one HIV protease inhibitor, and a process for preparing same.

The virus causing acquired immunodeficiency syndrome (AIDS) is known by different names, including T-lymphocyte virus III (HTLV-III) or lymphadenopathy-associated virus (LAV) or AIDS-related virus (ARV) or human immunodeficiency virus (HIV). Up until now, two distinct families have been identified, i. e., HIV-1 and HIV-2.

One of the critical pathways in a retroviral life cycle is the processing of polyprotein precursors by aspartic protease. For instance with the HIV virus the gag-pol protein is processed by HIV protease. The correct processing of the precursor polyproteins by the aspartic protease is required for the assembly of infectious virions, thus making the aspartic protease an attractive target for antiviral therapy. In particular for HIV treatment, the HIV protease is an attractive target.

A measure of the potential usefulness of an oral dosage form of a pharmaceutical agent is the bioavailability observed after oral administration of the dosage form. Various factors can affect the bioavailability of a drug when administered orally. These factors include aqueous solubility, drug absorption throughout the gastrointestinal tract, dosage strength and first pass effect. Aqueous solubility is one of the most important of these factors. Unfortunately, HIV protease inhibiting compounds typically are characterized by having poor aqueous solubility.

For a variety of reasons, such as patient compliance and taste masking, a solid dosage form is usually preferred over a liquid dosage form. In most instances however, oral solid dosage forms of a drug provide a lower bioavailability than oral solutions of the drug.

There have been attempts to improve the bioavailability provided by solid dosage forms by forming solid solutions of the drug. The term "solid solution" defines a system in a solid state wherein the drug is molecularly dispersed throughout a matrix such that the system is chemically and physically uniform or homogenous throughout. Solid solutions are preferred physical systems because the components therein readily form liquid solutions when contacted with a liquid medium such as gastric juice. The ease of dissolution may be attributed at least in part to the fact that the energy required for dissolution of the components from a solid solution is less than that required for the dissolution of the components from a crystalline or microcrystalline solid phase. If, however, the drug absorption in the gastrointestinal tract is slow the drug released from the solid solution may result in a high supersaturation and precipitate in the aqueous fluids of the gastrointestinal tract.

There is a continuing need for the development of improved oral solid dosage forms for HIV protease inhibitors which have suitable oral bioavailability and stability and which do not necessitate high vehicle volumes.

WO 01/034119 discloses a pharmaceutical composition comprising a solid dispersion of ritonavir.

The present invention provides a solid pharmaceutical dosage form according to claim 1 comprising a solid dispersion of at least one HIV protease inhibitor in at least one pharmaceutically acceptable water-soluble polymer and at least one pharmaceutically acceptable surfactant. In one embodiment, the pharmaceutically accepted water-soluble polymer has a glass transition temperatue (Tg) of at least about 50 °C.

The term "solid dispersion" defines a system in a solid state (as opposed to a liquid or gaseous state) comprising at least two components, wherein one component is dispersed evenly throughout the other component or components. For example, the active ingredient or combination of active ingredients is dispersed in a matrix comprised of the pharmaceutically acceptable water-soluble polymer(s) and pharmaceutically acceptable surfactant(s). The term "solid dispersion" encompasses systems having small particles, typically of less than 1 µm in diameter, of one phase dispersed in another phase. When said dispersion of the components is such that the system is chemically and physically uniform or homogenous throughout or consists of one phase (as defined in thermodynamics), such a solid dispersion will be called a "solid solution" or a "glassy solution". A glassy solution is a homogeneous, glassy system in which a solute is dissolved in a glassy solvent. Glassy solutions and solid solutions of HIV protease inhibitors are preferred physical systems. These systems do not contain any significant amounts of active ingredients in their crystalline or microcrystalline state, as evidenced by thermal analysis (DSC) or X-ray diffraction analysis (WAXS).

In one embodiment of the present invention, the pharmaceutical dosage form is comprising from about 5 to about 30 % by weight of the total dosage form (preferably from about 10 to about 25 % by weight of the total dosage form) of an HIV protease inhibitor or a combination of HIV protease inhibitors, from about 50 to about 85 % by weight of the total dosage form (preferably from about 60 to about 80 % by weight of the total dosage form) of a water-soluble polymer (or any combination of such polymers), from about 2 to about 20 % by weight of the total dosage form (preferably from about 3 to about 15 % by weight of the total dosage form) of the surfactant (or combination of surfactants), and from about 0 to about 15 % by weight of the total dosage form of additives.

HIV protease inhibiting compounds suitable for use in the present invention include for example, but are not limited thereto:
(2S,3S,5S)-5-(N-(N-((N-methyl-N-((2-isopropyl-4-thiazolyl)methyl)amino)carbonyl)-L-valinyl)amino-2-(N-((5-thiazolyl)methoxy-carbonyl)-amino)-amino-1,6-diphenyl-3hydroxyhexane (ritonavir);
(2S,3S,5S)-2-(2,6-Dimethylphenoxyacetyl)amino-3-hydroxy-5-[2S-(1-tetrahydro-pyrimid-2-onyl)-3-methylbutanoyl]-amino-1,6-diphenylhexane (ABT-378; lopinavir);
N-(2(R)-hydroxy-1(S)-indanyl)-2(R)-phenylmethyl-4(S)-hydroxy-5-(1-(4-(3-pyridyl-methyl)-2(S)-N'-(t-butylcarboxamido)-piperazinyl))-pentaneamide (indinavir);
N-tert-butyl-decahydro-2-2(R)-hydroxy-4-phenyl-3(S)-[[N-(2-quinolylcarbonyl)-L-asparaginyl]amino]butyl]-(4aS,8aS)-isoquinoline-3(S)-carboxamide (saquinavir);
5(S)-Boc-amino-4(S)-hydroxy-6-phenyl-2(R)phenylmethylhexanoyl-(L)-Val-(L)-Phe-morpholin-4-ylamide;
1-Naphthoxyacetyl-beta-methylthio-Ala-(2S,3S)3-amino-2-hydroxy-4-butanoyl-1,3-thiazolidine-4t-butylamide;
5-isoquinolinoxyacetyl-beta methylthio-Ala-(2S,3S)-3amino-2-hydroxy-4-butanoyl-1,3-thiazolidine-4-tbutylamide;
[1S-[1R-(R-),2S*])-N¹ [3-[[[(1,1-dimethylethyl)amino]carbonyl](2-methylpropyl)amino]-2hydroxy-1-(phenylmethyl)propyl]-2-[(2-quinolinylcarbonyl)amino]-butanediamide;
amprenavir (VX-478); DMP-323; DMP-450; AG1343 (nelfinavir);
atazanavir (BMS 232,632);
tipranavir;
palinavir;
TMC-114;
RO033-4649;
fosamprenavir (GW433908);
P-1946;
BMS 186,318; SC-55389a; BILA 1096 BS; and U-140690, or combinations thereof,

Ritonavir (Abbott Laboratories, Abbott Park, IL, USA) is an HIV protease inhibitor which is formulated into the dosage form of the invention. This and other compounds as well as methods for preparing same are disclosed in U. S. Patent Nos. 5,542,206 and 5,648,497, In an embodiment, the present invention provides a dosage form wherein said HIV protease inhibitor is ritonavir or a combination of ritonavir and at least one other HIV protease inhibitor, the dosage form showing a dose-adjusted AUC of ritonavir plasma concentration in dogs of at least about 9 µg.h/ml/100 mg.

In an embodiment, lopinavir (Abbott Laboratories, Abbott Park, IL, USA) is an HIV protease inhibitor which may be formulated into the dosage form of the invention. This and other compounds, as well as methods for preparing same, are identified in U. S. Patent No. 3,914,332.

In yet another embodiment, nelfinavir mesylate (marketed under the tradename Viracept by Agouron Pharmaceuticals, Inc. in La Jolla, CA) is an HIV protease inhibitor which may be formulated into the dosage form of the invention.

The dosage forms of the present invention exhibit a release and absorption behaviour that is characterized by high attainable AUC, high attainable Cₘₐₓ (maximum plasma concentration), and low Tₘₐₓ (time to reach maximum plasma concentration).

In still another embodiment, the present invention provides a dosage form wherein said HIV protease inhibitor is a combination of ritonavir and lopinavir, the dosage form showing a dose-adjusted AUC of ritonavir plasma concentration in dogs of at least about 9 µg.h/ml/100 mg and a dose-adjusted AUC of lopinavir plasma concentration of at least about 20 µg.h/ml/100 mg (preferably at least about 22,5 µg.h/ml/100 mg, most preferred at least about 35 µg.h/ml/100 mg).

The term "AUC" means "Area Under the Curve" and is used in its normal meaning, i. e. as the area under the plasma concentration-time curve from 0 to 24 hours, where the dosage form has been administered orally to dogs (beagle) under non-fasting conditions. "Non-fasting condition" means that the dogs receive a nutritionally balanced daily ration during the pre-test period and the whole test period. The AUC has units of concentration times time. Once the experimental concentration-time points have been determined, the AUC may conveniently be calculated; e.g. by a computer program or by the trapezoidal method. All AUC data herein were dose adjusted to the 100 mg dose level. For the purposes herein, the AUC is determined within a dose range where the AUC increases proportionally with dose. Administration of 50 mg ritonavir or 200 mg lopinavir, respectively, to dogs is considered suitable for determining the AUC values as used herein.

The dosage forms according to the invention are characterized by an excellent stability and, in particular, exhibit high resistance against recrystallization or decomposition of the active ingredient(s). Thus, upon storage for 6 weeks at 40°C and 75% humidity (e.g., when kept in high density polyethylene (HDPE) bottles without desiccant), the dosage forms according to the present invention usually do not exhibit any sign of crystallinity (as evidenced by DSC or WAXS analysis) and contain at least about 98 % of the initial active ingredient content (as evidenced by HPLC analysis).

The term "pharmaceutically acceptable surfactant" as used herein refers to a pharmaceutically acceptable non-ionic surfactant. The dosage form of the invention comprises a surfactant having an hydrophilic lipophilic balance (HLB) value of from 4 to 10, preferably from about 7 to about 9. The HLB system (Fiedler, H.B., Encyclopedia of Excipients, 5th ed., Aulendorf: ECV-Editio-Cantor-Verlag (2002)) attributes numeric values to surfactants, with lipophilic substances receiving lower HLB values und hydrophilic substances receiving higher HLB values. Surfactants having an HLB value of from 4 to 10 suitable for use in the present invention include for example, but are not limited thereto;
polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether; polyoxyethylene (4) nonylphenyl ether, polyoxyethylene (3) octylphenyl ether;
polyethylene glycol fatty acid esters, e.g. PBG-200 monolaurate, PEG-200 dilaurate, PEG-300 dilaurate, PEG-400 dilaurate, PEG-300 distearate, PEG-300 dioleate;
alkylene glycol fatty acid mono esters, e.g. propylene glycol monolaurate (Lauroglycol®);
sucrose fatty acid esters, e.g. sucrose monostearate, sucrose distearate, sucrose monolaurate, sucrose dilaurate; or
sorbitan fatty acid mono esters such as sorbitan mono laurate (Span® 20), sorbitan monooleate, sorbitan monopalmitate (Span® 40), or sorbitan stearate, or
mixtures of one or more thereof.

The sorbitan mono fatty acid esters are preferred, with sorbitan mono laurate and sorbitan monopalmitate being particularly preferred.

Besides the surfactant having an HLB value of from 4 to 10, the dosage form may comprise additional pharmaceutically acceptable surfactants such as polyoxyethylene castor oil derivates, e.g. polyoxyethyleneglycerol triricinoleate or polyoxyl 35 castor oil (Cremophor® EL; BASF Corp.) or polyoxyethyleneglycerol oxystearate such as polyethylenglycol 40 hydrogenated castor oil (Cremophor® RH 40) or polyethylenglycol 60 hydrogenated castor oil (Cremophor® RH 60); or block copolymers of ethylene oxide and propylene oxide, also known as polyoxyethylene polyoxypropylene block copolymers or polyoxyethylene polypropyleneglycol, such as Poloxamer® 124, Poloxamer® 188, Poloxamer® 237, Poloxamer® 388, Poloxamer® 407 (BASF Wyandotte Corp.); or a mono fatty acid ester of polyoxyethylene (20) sorbitan, e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), polyoxyethylene (20) sorbitan monolaurate (Tween® 20),

Where such additional surfactants are used, the surfactant having an HLB value of from 4 to 10 generally accounts for at least about 50 % by weight, preferably at least about 60 % by weight, of the total amount of surfactant used.

In one embodiment of the present invention, the water-soluble polymer employed has a Tg of at least about 50 °C, preferably at least about 60 °C, most preferred from about 80 °C to about 180 °C. Methods for determining Tg values of the organic polymers are described in "Introduction to Physical Polymer Science", 2nd Edition by L.H. Sperling, published by John Wiley & Sons, Inc., 1992. The Tg value can be calculated as the weighted sum of the Tg values for homopolymers derived from each of the individual monomers, i.e., that make up the polymer: Tg = E Wᵢ Xᵢ where W is the weight percent of monomer i in the organic polymer, and X is the Tg value for the homopolymer derived from monomer i. Tg values for the homopolymers may be taken from "Polymer Handbook", 2nd Edition by J. Brandrup and E.H. Immergut, Editors, published by John Wiley & Sons, Inc., 1975.

Water-soluble polymers having a Tg as defined above allow for the preparation of solid dispersions that are mechanically stable and, within ordinary temperature ranges, sufficiently temperature stable so that the solid dispersions may be used as dosage forms without further processing or be compacted to tablets with only a small amount of tabletting aids.

The water-soluble polymer comprised in the dosage form is a polymer that preferably has an apparent viscosity, when dissolved at 20 °C in an aqueous solution at 2 % (w/v), of about 1 to about 5000 mPa.s. more preferably of about 1 to about 700 mPa.s, and most preferred of about 5 to about 100 mPa.s. Water-soluble polymers suitable for use in the present invention include for example, but are not limited thereto:
homopolymers and copolymers of N-vinyl lactams, escpecially homopolymers and copolymers of N-vinyl pyrrolidone, e.g. polyvinylpyrrolidone (PVP), copolymers of N-vinyl pyrrolidone and vinyl acetate or vinyl propionate,
cellulose esters and cellulose ethers, in particular methylcellulose and ethylcellulose, hydroxyalkylcelluloses, in particular hydroxypropylcellulose, hydroxyalkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate;
high molecular polyalkylene oxides such as polyethylene oxide and polypropylene oxide and copolymers of ethylene oxide and propylene oxide,
polyacrylates and polymethacrylates such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), poly(hydroxyalkyl methacrylates),
polyacrylamides,
vinyl acetate polymers such as copolymers of vinyl acetate and crotonic acid, partially hydrolyzed polyvinyl acetate (also referred to as partially saponified "polyvinyl alcohol"),
polyvinyl alcohol,
oligo- and polysaccharides such as carrageenans, galactomannans and xanthan gum, or mixtures of one or more thereof.

Of these, homopolymers or copolymers of N-vinyl pyrrolidone, in particular a copolymer of N-vinyl pyrrolidone and vinyl acetate, are preferred. A particularly preferred polymer is a copolymer of about 60 % by weight of the copolymer, N-vinyl pyrrolidone and about 40 % by weight of the copolymer, vinyl acetate.

The dosage forms of the invention may contain at least one conventional additive, such as flow regulators, lubricants, bulking agents (fillers) and disintegrants. In general, the additive is contained in an amount of about 0.01 to about 15 % by weight relative to the weight of the dosage form.

Various methods can be used for manufacturing the solid dosage forms according to the invention. These methods comprise the preparation of a solid solution of the HIV protease inhibitor or the combination of HIV protease inhibitors in a matrix of the water-soluble polymer and the surfactant, and shaping into the required tablet form. Alternatively, the solid solution product may be subdivided to granules, e.g. by grinding or milling, and the granules may subsequently be compacted to tablets.

Various techniques exist for preparing solid solutions including melt-extrusion, spray-drying and solution-evaporation with melt-extrusion being preferred.

The melt-extrusion process comprises the steps of preparing a homogeneous melt of the HIV protease inhibitor or the combination of HIV protease inhibitors, the water-soluble polymer and the surfactant, and cooling the melt until it solidifies. "Melting" means a transition into a liquid or rubbery state in which it is possible for one component to get embedded homogeneously in the other. Typically, one component will melt and the other components will dissolve in the melt thus forming a solution. Melting usually involves heating above the softening point of the water-soluble polymer. The preparation of the melt can take place in a variety of ways. The mixing of the components can take place before, during or after the formation of the melt. For example, the components can be mixed first and then melted or be simultaneously mixed and melted. Usually, the melt is homogenized in order to disperse the active ingredients efficiently. Also, it may be convenient first to melt the water-soluble polymer and then to mix in and homogenize the active ingredients.

Usually, the melt temperature is in the range of about 70 to about 250 °C, preferably from about 80 to about 180 °C, most preferred from about 100 to about 140 °C.

The active ingredients can be employed as such or as a solution or dispersion in a suitable solvent such as alcohols, aliphatic hydrocarbons or esters. Another solvent which can be used is liquid carbon dioxide. The solvent is removed, e.g. evaporated, upon preparation of the melt.

Various additives may be included in the melt, for example flow regulators such as colloidal silica; lubricants, fillers, disintegrants, plasticizers, stabilizers such as antioxidants, light stabilizers, radical scavengers, stabilizers against microbial attack.

The melting and/or mixing takes place in an apparatus customary for this purpose. Particularly suitable ones are extruders or kneaders. Suitable extruders include single screw extruders, intermeshing screw extruders or else multiscrew extruders, preferably twin screw extruders, which can be corotating or counterrotating and, optionally, be equipped with kneading disks. It will be appreciated that the working temperatures will also be determined by the kind of extruder or the kind of configuration within the extruder that is used. Part of the energy needed to melt, mix and dissolve the components in the extruder can be provided by heating elements. However, the friction and shearing of the material in the extruder may also provide a substantial amount of energy to the mixture and aid in the formation of a homogeneous melt of the components.

The melt ranges from pasty to viscous. Shaping of the extrudate conveniently is carried out by a calender with two counter-rotating rollers with mutually matching depressions on their surface. A broad range of tablet forms can be attained by using rollers with different forms of depressions. Alternatively, the extrudate is cut into pieces, either before (hot-cut) or after solidification (cold-cut).

Optionally, the resulting solid solution product is milled or ground to granules. The granules may then be compacted. Compacting means a process whereby a powder mass comprising the granules is densified under high pressure in order to obtain a compact with low porosity, e.g. a tablet. Compression of the powder mass is usually done in a tablet press, more specifically in a steel die between two moving punches. Where a solid dosage form of the invention comprises a combination of more than one HIV protease inhibitor (or a combination of an HIV protease inhibitor with one or more other active ingredients) it is of course possible to separately prepare solid solution products of the individual active ingredients and to blend the milled or ground products before compacting.

At least one additive selected from flow regulators, disintegrants, bulking agents (fillers) and lubricants is preferably used in compacting the granules. Disintegrants promote a rapid disintegration of the compact in the stomach and keeps the granules which are liberated separate from one another. Suitable disintegrants are crosslinked polymers such as crosslinked polyvinyl pyrrolidone and crosslinked sodium carboxymethylcellulose. Suitable bulking agents (also referred to as "fillers") are selected from lactose, calcium hydrogenphosphate, microcrystalline cellulose (Avicell®), silicates, in particular silicium dioxide, magnesium oxide, talc, potato or corn starch, isomalt, polyvinyl alcohol.

Suitable flow regulators are selected from highly dispersed silica (Aerosil®), and animal or vegetable fats or waxes.

A lubricant is preferably used in compacting the granules. Suitable lubricants are selected from polyethylene glycol (e.g., having a Mw of from 1000 to 6000), magnesium and calcium stearates, sodium stearyl fumarate, and the like.

Various other additives may be used, for example dyes such as azo dyes, organic or inorganic pigments such as aluminium oxide or titanium dioxide, or dyes of natural origin; stabilizers such as antioxidants, light stabilizers, radical scavengers, stabilizers against microbial attack.

Dosage forms according to the invention may be provided as dosage forms consisting of several layers, for example laminated or multilayer tablets. They can be in open or closed form. "Closed dosage forms" are those in which one layer is completely surrounded by at least one other layer. Multilayer forms have the advantage that two active ingredients which are incompatible with one another can be processed, or that the release characteristics of the active ingredient(s) can be controlled. For example, it is possible to provide an initial dose by including an active ingredient in one of the outer layers, and a maintenance dose by including the active ingredient in the inner layer(s). Multilayer tablets types may be produced by compressing two or more layers of granules. Alternatively, multilayer dosage forms may be produced by a process known as "coextrusion". In essence, the process comprises preperation of at least two different melt compositions as explained above, and passing these molten compositions into a joint coextrusion die. The shape of the coextrusion die depends on the required drug form. For example, dies with a plain die gap, called slot dies, and dies with an annular slit are suitable.

In order to faciliate the intake of such a dosage form by a mammal, it is advantageous to give the dosage form an appropriate shape. Large tablets that can be swallowed comfortably are therefore preferably elongated rather than round in shape.

A film coat on the tablet further contributes to the ease with which it can be swallowed. A film coat also improves taste and provides an elegant appearance. If desired, the film-coat may be an enteric coat. The film-coat usually includes a polymeric film-forming material such as hydroxypropyl methylcellulose, hydroxypropylcellulose, and acrylate or methacrylate copolymers. Besides a film-forming polymer, the film-coat may further comprise a plasticizer, e.g. polyethylene glycol, a surfactant, e.g. a Tween® type, and optionally a pigment, e.g. titanium dioxide or iron oxides. The film-coating may also comprise talc as anti-adhesive. The film coat usually accounts for less than about 5 % by weight of the dosage form.

The exact dose and frequency of administration depends on the particular condition being treated, the age, weight and general physical condition of the particular patient as well as other medication the individual may be taking, as is well known to those skilled in the art.

Exemplary compositions of the present invention for combined administration of ritonavir/ lopinavir are shown below in Table 1, and the values are % by weight.

**Table 1.**

| | | | |
|---|---|---|---|
| Ritonavir | 18 - 22.5 in total | 4.17 | 4.17 |
| Lopinavir | | 16.67 | 16.67 |
| Copovidone (N-vinyl pyrrolidone/vinyl acetate copolymer 60:40) | 65 -75 | 71.16 | 70.12 |
| Span 20 (Sorbitan monolaurate) | 4 -10 | 7.0 | 5.02 |
| Cremophor RH40 (polyoxyethyleneglycerol oxystearate) | 0 -10 | - | 3.02 |
| Colloidal silica | 0-3 | 1.0 | 1.0 |

Exemplary compositions for administration of ritonavir only are shown below in Table 2. The values are % by weight.

| | | |
|---|---|---|
| Ritonavir | 18 -22.5 | 20.8 |
| Lopinavir | - | - |
| Copovidone (N-vinyl pyrrolidone/vinyl acetate copolymer 60:40) | 60 -75 | 63.15 |
| Span 20 (Sorbitan monolaurate) | 5 -15 in total | - |
| Cremophor RH40 (polyoxyethyleneglycerol oxystearate) | | 10.00 |
| PEG 6000 | 0 -8 | 5.00 |
| Colloidal silica | 0 -3 | 1.04 |

The above compositions are processed by melt extrusion. The resulting extrudates may be used as such or milled and compressed into tablets, preferably by the use of suitable tabletting aids such as sodium stearyl fumarate, colloidal silica, lactose, isomalt, calcium silicate, and magnesium stearate, cellulose or calcium hydrogenphosphate.

The following examples will serve to further illustrate the invention without limiting it.

### Protocol for the oral bioavailability studies

Dogs (beagle dogs, mixed sexes, weighing approximately 10 kg) received a balanced diet with 27 % fat and were permitted water ad libitum. Each dog received a 100 µg/kg subcutaneous dose of histamine approximately 30 minutes prior to dosing. A single dose corresponding to about 200 mg lopinavir, about 50 mg ritonavir, or about 200 mg lopinavir and about 50 mg ritonavir, respectively, was administered to each dog. The dose was followed by approximately 10 milliliters of water. Blood samples were obtained from each animal prior to dosing and 0.25, 0.5, 1.0, 1.5, 2, 3, 4, 6, 8, 10, 12 and 24 hours after drug administration. The plasma was separated from the red cells by centrifugation and frozen (-30 °C) until analysis. Concentrations of HIV protease inhibitors were determined by reverse phase HPLC with low wavelength UV detection following liquid-liquid extraction of the plasma samples. The area under the curve (AUC) was calculated by the trapezoidal method over the time course of the study. Each dosage form was evaluated in a group containing 8 dogs; the values reported are averages for each group of dogs.

### Comparative example

Copovidone (N-vinyl pyrrolidone/vinyl acetate copolymer 60:40; 78.17 parts by weight) was mixed with ritonavir (4.16 parts by weight), lopinavir (16.67 parts by weight) and colloidal silica (1.0 part by weight). The powdery mixture was then fed into a twin-screw extruder (screw diameter 18 mm) at a rate of 2.0 kg/h and a melt temperature of 133 °C. The clear, fully transparent melt was fed to a calender with two counter-rotating rollers having mutually matching cavities on their surfaces. Tablets of 1080 mg were thus obtained. DSC and WAXS analysis did not reveal any evidence of crystalline drug material in the formulation.

The dose-adjusted AUC in dogs was 0.52 µg.h/ml/100 mg for ritonavir and 4.54 µg.h/ml/100 mg for lopinavir. This example shows that solid solutions of HIV protease inhibitors without added surfactant yield a very poor bioavailabilty.

### Example 1 (reference)

Copovidone (N-vinyl pyrrolidone/vinyl acetate copolymer 60:40; 68.17 parts by weight) was blended with Cremophor RH40 (polyoxyethyleneglycerol oxystearate; 10.00 parts by weight) in a Diosna high-shear mixer. The resulting granules were mixed with ritonavir (4.17 parts by weight), lopinavir (16.67 parts by weight) and colloidal silica (1.00 parts by weight). The powdery mixture was then fed into a Leistritz Micro 18 twin-screw extruder at a rate of 2.3 kg/h and a melt temperature of 126 °C. The extrudate was cut into pieces and allowed to solidify. The extruded pieces were milled using a high impact universal mill. The milled material (86.49 parts by weight) was blended in a bin blender with lactose monohydrate (6.00 parts by weight), crosslinked PVP (6.00 parts by weight), colloidal silica (1.00 part by weight) and magnesium stearate (0.51 parts by weight). The powdery blend was compressed to tablets of 1378.0 mg on a Fette E 1 single punch tablet press. The tablets were then film-coated in a coating pan by spraying an aqueous dispersion for film coating (Opadry, available from Colorcon) at a temperature of 60 °C.

The dose-adjusted AUC in dogs was 0.60 µg.h/ml/100 mg for ritonavir and 7.43 µg.h/ml/100 mg for lopinavir. This example shows that inclusion of a surfactant into solid solutions of HIV protease inhibitors improves the bioavailabilty attained.

### Example 2

Copovidone (N-vinyl pyrrolidone/vinyl acetate copolymer 60:40; 853.8 parts by weight) was blended with Span 20 (Sorbitan monolaurate; 83.9 parts by weight) in a Diosna high-shear mixer. The resulting granules were mixed with ritonavir (50 parts by weight), lopinavir (200 parts by weight) and colloidal silica (12 parts by weight). The powdery mixture was then fed into a twin-screw extruder (screw diameter 18 mm) at a rate of 2.1 kg/h and a melt temperature of 119 °C. The extrudate was fed to a calender with two counter-rotating rollers having mutually matching cavities on their surfaces. Tablets of 1120 mg were thus obtained.

The dose-adjusted AUC in dogs was 10.88 µg.h/ml/100 mg for ritonavir and 51.2 µg.h/ml/100 mg for lopinavir. This example shows that inclusion of a surfactant having an HLB of 4 to 10 into solid solutions of HIV protease inhibitors markedly improves the bioavailability attained.

### Example 3

Example 2 was repeated, however, the extrudate was cut into pieces and allowed to solidify. The extruded pieces were milled to a particle size of about 250 µm, using a high impact universal mill. The milled material was blended in a bin blender with sodium stearyl fumarate (12.3 parts by weight) and colloidal silica (8.0 parts by weight) for 20 min. The powdery blend was compressed on a rotary tablet machine with 3 punches (6500 tablets/h). The tablets were then film-coated in a coating pan by spraying an aqueous dispersion for film coating (Opadry) at a temperature of 60 °C.

The dose-adjusted AUC in dogs was 14.24 µg.h/ml/100 mg for ritonavir and 52.2 µg.h/ml/100 mg for lopinavir.

### Example 4

Copovidone (N-vinyl pyrrolidone/vinyl acetate copolymer 60:40; 841.3 parts by weight) was blended with Cremophor RH40 (polyoxyethyleneglycerol oxystearate; 36.2 parts by weight), Span 20 (Sorbitan monolaurate; 60.2 parts by weight) in a Diosna high-shear mixer. The resulting granules were mixed with ritonavir (50 parts by weight), lopinavir (200 parts by weight) and colloidal silica (12 parts by weight). The powdery mixture was then fed into a twin-screw extruder (screw diameter 18 mm) at a rate of 2.1 kg/h and a melt temperature of 114 °C. The extrudate was fed to a calender with two counter-rotating rollers having mutually matching cavities on their surfaces. Tablets of 1120 mg were thus obtained.

The dose-adjusted AUC in dogs was 10.96 µg.h/ml/100 mg for ritonavir and 46.5 µg.h/ml/100 mg for lopinavir. This example shows that a combination of a surfactant having an HLB of 4 to 10 and a further surfactant can successfully be used.

### Example 5

Example 4 was repeated, however, the extrudate was cut into pieces and allowed to solidify. The extruded pieces were milled to a particle size of about 250 µm, using a high impact universal mill. The milled material was blended in a bin blender with sodium stearylfumarate (13.9 parts by weight), colloidal silica (7.0 parts by weight), isomalt DC100 (159.4 parts by weight) and calcium silicate (7.0 parts by weight) for 20 min. The blend was compressed and film-coated as described in example 1.

The dose-adjusted AUC in dogs was 10.38 µg.h/ml/100 mg for ritonavir and 42.7 µg.h/ml/100 mg for lopinavir.

### Example 6 (reference)

Copovidone (N-vinyl pyrrolidone/vinyl acetate copolymer 60:40; 683.3 parts by weight) was blended with Span 40 (sorbitan monopalmitate; 67.2 parts by weight) in a Diosna high-shear mixer. The resulting granules were mixed with lopinavir (200 parts by weight) and colloidal silica (9.6 parts by weight). The powdery mixture was then fed into a twin-screw extruder (screw diameter 18 mm) at a rate of 2.1 kg/h and a melt temperature of 119 °C. The extrudate was cut into pieces and allowed to solidify. The extruded pieces were milled using a high impact universal mill. The milled material was blended in a bin blender with sodium stearylfumarate (7.9 parts by weight), colloidal silica (11.3 parts by weight), isomalt DC100 (129.1 parts by weight) and sodium dodecyl sulfate (15.6 parts by weight). The blend was compressed and film-coated as described in example 1.

Tablets corresponding to 200 mg lopinavir were coadministered to dogs together with 50 mg ritonavir. The dose-adjusted AUC of lopinavir was 38.8 µg.h/ml/100 mg.

### Example 7 (reference)

Copovidone (N-vinyl pyrrolidone/vinyl acetate copolymer 60:40; 151.5 parts by weight) was blended with Cremophor RH40 (24 parts by weight) and PEG 6000 (12 parts by weight) in a Diosna high-shear mixer. The resulting granules were mixed with ritonavir (50 parts by weight) and colloidal silica (2.4 parts by weight). The powdery mixture was then fed into a twin-screw extruder and was.melt-extruded. The extrudate was cut into pieces and allowed to solidify. The extruded pieces were milled using a high impact universal mill. The milled material was blended in a bin blender with colloidal silica (1.4 parts by weight), isomalt DC100 (31.9 parts by weight) and calcium silicate (4.2 parts by weight). The blend was compressed and film-coated as described in example 1.

The dose-adjusted AUC in dogs was 9.98 µg.h/ml/100 mg.

The following numbered embodiments are preferred instances of the present disclosure:
1. A solid pharmaceutical dosage form which comprises a solid dispersion of at least one HIV protease inhibitor and at least one pharmaceutically acceptable water-soluble polymer and at least one pharmaceutically acceptable surfactant, said pharmaceutically acceptable water-soluble polymer having a Tg of at least about °50 C.
2. The dosage form of the above embodiment 1 comprising a glassy solution or solid solution of said HIV protease inhibitor.
3. The dosage form of the above embodiment 1, wherein said pharmaceutically acceptable surfactant has an HLB value of from about 4 to about 10.
4. The dosage form of the above embodiment 1, wherein said pharmaceutically acceptable surfactant is a combination of at least one pharmaceutically acceptable surfactant having an HLB value of from about 4 to about 10 and at least one further pharmaceutically acceptable surfactant.
5. The dosage form of the above embodiment 1 wherein said pharmaceutically acceptable surfactant is a sorbitan fatty acid ester.
6. The dosage form of the above embodiment 1 which comprises, relative to the weight of the dosage form, from about 5 to about 30 % by weight of said HIV protease inhibitor, from about 50 to about 85 % by weight of said water-soluble polymer, from about 2 to about 20 % by weight of said surfactant, and from about 0 to about 15 % by weight of additives.
7. The dosage form of the above embodiment 1, wherein said HIV protease inhibitor is selected from the group consisting of:
   2S,3S,5S)-5-(N-(N-((N-methyl-N-((2-isopropyl-4-thiazolyl)methyl)amino)carbonyl)-L-valinyl)amino-2-(N-((5-thiazolyl)methoxy-carbonyl)-amino)-amino-1,6-diphenyl-3-hydroxyhexane (ritonavir);
   (2S,3S,5S)-2-(2,6-Dimethylphenoxyacetyl)amino-3-hydxoxy-5-[2S-(1-tetrahydro-pyrimid-2-onyl)-3-methylbutanoyl]amino-1,6-diphenylhexane (lopinavir);
   N-(2(R)-hydroxy-1(S)-indanyl)-2(R)-phenylmethyl-4(S)-hydroxy-5-(1-(4-(3-pyridylmethyl)-2(S)-N'-(t-butylcarboxamido)-piperaziayl))-pentaneamide (indinavir);
   N-tert-butyl-decahydro-2-[2(R)-hydroxy-4-phenyl-3(S)-[[N-(2-quinolylcarbonyl)-L-asparaginyl]amino]butyl]-(4aS,8aS)-isoquinoline-3(S)-carboxamide (saquinavir);
   5(S)-Boc-amino-4(S)-hydroxy-6-phenyl-2(R)phenylmethylhexanoyl-(L)-Val-(L)-Phe-morpholin-4-ylamide;
   1-Naphthoxyacetyl-beta-methylthio-Ala-(2S,3S)3-amino-2-hydroxy-4-butanoyl 1,3-thiazolidine-4t-butylamide;
   5-isoquino linoxyacetyl-beta-methylthio-Ala-(2S,3S)-3-amino-2-hydroxy-4-butanoyl-1,3-thiazolidine-4-t-butylamide;
   [1S-[1R-(R-),2S*])-N'-[3-[[[(1,1-dimethylethyl)amino] carbonyl](2-methylpropyl)amino]-2hydroxy-1-(phenylmethyl)propyl]-2-[(2-quinolinylcarbonyl)amino]-butanediamide;
   amprenavir (VX-478); DMP-323; DMP-450; AG1343 (nelfinavir);
   atazanavir (BMS 232,632)
   tipranavir
   palinavir
   TMC-114
   RO033-4649
   fosamprenavir (GW433908)
   P-1946,
   BMS 186,318; SC-55389a; BILA 1096 BS; U-140690,
   or combinations thereof.
8. The dosage form of the above embodiment 1 wherein said HIV protease inhibitor is (2S,3S,5S)-5-(N-(N-((N-methyl-N-((2-isopropyl-4-thiazolyl)methyl)amino)carbonyl)-L-valinyl)amino-2-(N-((5-thiazolyl)methoxycarbonyl)-amino)amino-1,6-diphenyl-3-hydroxyhexane (ritonavir).
9. The dosage form of the above embodiment 8 which shows a dose-adjusted AUC, in dogs under non-fasting conditions, of ritonavir plasma concentration of at least 9 µg.h/ml/100 mg.
10. The dosage form of the above embodiment 1 wherein said HIV protease inhibitor is (2S,3S,5S)-2-(2,6-Dimethylphenoxyacetyl)-amino-3-hydroxy-5-[2S-(1-tetrahydropyrimid-2-onyl)-3-methyl-butanoyl] amino-1,6-diphenylhexane (lopinavir).
11. The dosage form of the above embodiment 10 which shows a dose-adjusted AUC, in dogs under non- fasting conditions, of lopinavir plasma concentration of at least about 20 µg.h/ml/100 mg.
12. The dosage form of the above embodiment 1 wherein said HIV protease inhibitor is a combination of (2S,3S,5S)-5-(N-(N-((N-methyl-N-((2-isopropyl-4-thiazolyl) methyl)amino)carbonyl)-L-valinyl)amino-2-(N-((5-thiazolyl)methoxy-carbonyl)-amino)-amino-1,6-diphenyl-3-hydroxyhexane (ritonavir) and (2S, 3S,5S)-2-(2,6-Dimethylphenoxyacetyl)amino-3-hydroxy-5-[2S-(1-tetrahydropyrimid-2-onyl)-3-methylbutanoyl]amino-1,6-diphenylhexane (lopinavir).
13. The dosage form of the above embodiment 12 which shows a dose-adjusted AUC, in dogs under non- fasting conditions, of ritonavir plasma concentration of at least about 9 µg.h/ml/100 mg and a dose-adjusted AUC of lopinavir plasma concentration of at least about 20 µg. h/ml/100 mg.
14. The solid dosage form of the above embodiment 1 wherein said water-soluble polymer has a Tg of from about 80 to about 180 °C.
15. The solid dosage form of the above embodiment 1 wherein said water-soluble polymer is a homopolymer or copolymer of N-vinyl pyrrolidone.
16. The solid dosage form of the above embodiment 1 wherein said water-soluble polymer is a copolymer of N-vinyl pyrrolidone and vinyl acetate.
17. The solid dosage form of the above embodiment 1 containing at least one additive selected from flow regulators, disintegrants, bulking agents and lubricants.
18. The solid dosage form of the above embodiment 1 which contains, upon storage for about 6 weeks at about 40 °C and about 75% humidity, at least about 98% of the initial content of HIV protease inhibitor.
19. A method of preparing a solid-dosage-form of the above embodiment 1 which comprises:
   i. preparing a homogeneous melt of said HIV protease inhibitor (s), said water- soluble polymer (s) and said surfactant (s), and
   ii. allowing the melt to solidify to obtain a solid dispersion product.
20. The method of the above embodiment 19 additionally comprising grinding said solid dispersion product and compressing said solid dispersion product into a tablet.
21. A method of treating an HIV infection comprising administering the solid dosage form of the above embodiment 1 to a mammal in need of such treatment.
22. A solid pharmaceutical dosage form comprising,
   (2S,3S,5S)-5-(N-(N-((N-methyl-N-((2-isopropyl-4-thiazolyl)methyl)amino)carbonyl)-L-valinyl)amino-2-(N-((5-thiazolyl)methoxycarbonyl)-amino)-amino-1,6-diphenyl-3- hydroxyhexane (ritonavir);
   a homopolymer of N-vinyl pyrrolidone; and
   a sorbitan fatty acid ester.
23. The solid dosage form of the above embodiment 22 containing at least one additive selected from flow regulators, disintegrants, bulking agents and lubricants.
24. A solid pharmaceutical dosage form comprising,
   (2S,3S,5S)-2-(2,6-Dimethylphenoxyacetyl)amino-3-hydroxy-5- [2S-(1-tetrahydro-pyrimid-2-onyl)-3-methylbutanoyl] amino-1, 6-diphenylhexane (lopinavir);
   a copolymer of N-vinyl pyrrolidone; and
   a sorbitan fatty acid ester.
25. The solid dosage form of the above embodiment 24 containing at least one additive selected from flow regulators, disintegrants, bulking agents and lubricants.
26. A solid pharmaceutical dosage form comprising,
   (2S,3S,5S)-5-(N-(N-((N-methyl-N-((2-isopropyl-4-thiazolyl)methyl)amino)-carbonyl)-L-valinyl)amino-2-(N-((5-thiazolyl) methoxy-carbonyl)-amino)-amino-1,6-diphenyl-3- hydroxyhexane (ritonavir) and (2S,3S,5S)-2-(2,6-Dimethylphenoxyacetyl) amino-3-hydroxy-5-[2S-(1-tetrahydro-pyrimid-2-onyl)-3-methylbutanoyl] amino-1,6-diphenylhexane (lopinavir);
   a copolymer of N-vinyl pyrrolidone and vinyl acetate; and
   a sorbitan fatty acid ester.
27. The solid dosage form of the above embodiment 26 containing at least one additive selected from flow regulators, disintegrants, bulking agents and lubricants.
28. A solid pharmaceutical dosage form comprising,
   (2S,3S,5S)-5-(N-(N-((N-methyl-N-((2-isopropyl-4-thiazolyl)methyl)amino)carbonyl)-L-valinyl)amino-2-(N-((5-thiazolyl)methoxy-carbonyl)-amino)-amino-1,6-diphenyl-3-hydroxyhexane (ritonavir) from about 5 % to about 30 % by weight of the dosage form;
   a homopolymer of N-vinyl pyrrolidone from about 50 % to about 85 % by weight of the dosage form; and
   a sorbitan fatty acid ester from about 2 % to about 20 % by weight of the dosage form.
29. The solid dosage form of the above embodiment 28 containing at least one additive selected from flow regulators, disintegrants, bulking agents and lubricants.
30. The solid dosage form of the above embodiment 29 wherein the at least one additive is present in an amount from about 0 % to about 15 % by weight.
31. A solid pharmaceutical dosage form comprising,
   (2S,3S,5S)-2-(2,6-Dimethylphenoxyacetyl)amino-3-hydroxy-5-[2S-(1-tetrahydro-pyrimid-2-onyl)-3-methylbutanoyl]amino-1,6-diphenylhexane (lopinavir) from about 5 % to about 30 % by weight of the dosage form;
   a copolymer of N-vinyl pyrrolidone from about 50 % to about 85 % by weight of the dosage form; and
   a sorbitan fatty acid ester from about 2 % to about 20 % by weight of the dosage form.
32. The solid dosage form of the above embodiment 31 containing at least one additive selected from flow regulators, disintegrants, bulking agents and lubricants.
33. The solid dosage form of the above embodiment 32 wherein the at least one additive is present in an amount from about 0 % to about 15 % by weight.
34. A solid pharmaceutical dosage form comprising,
   (2S,3S,5S)-5-(N-(N-((N-methyl-N-((2-isopropyl-4-thiazolyl)methyl)amino)-carbonyl)-L-valinyl)amino-2-(N-((5-thiazolyl)methoxy-carbonyl)-amino)-amino-1,6-diphenyl-3-hydroxyhexane (ritonavir) and (2S,3S,5S)-2-(2,6-Dimethylphenoxyacetyl)amino-3-hydroxy-5-[2S-(1-tetrahydro-pyrimid-2-onyl)-3-methylbutanoyl]amino-1,6-diphenylhexane (lopinavir) present in an amount from about 5 % to about 30 % by weight of the dosage form;
   a copolymer of N-vinyl pyrrolidone and vinyl acetate from about 50 % to about 85 % by weight of the dosage form; and
   a sorbitan fatty acid ester from about 2 % to about 20 % by weight of the dosage form.
35. The solid dosage form of the above embodiment 34 containing at least one additive selected from flow regulators, disintegrants, bulking agents and lubricants.
36. The solid dosage form of the above embodiment 35 wherein the at least one additive is present in an amount from about 0 % to about 15 % by weight of the dosage form.
37. A method of treating an HIV infection comprising administering the solid dosage form of any one of the above embodiments 22-36 to a mammal in need of such treatment.

## Claims

1. A solid pharmaceutical dosage form which comprises a solid dispersion of at least one HIV protease inhibitor in at least one pharmaceutically acceptable water-soluble polymer and at least one pharmaceutically acceptable surfactant, wherein said dosage form comprises ritonavir and a surfactant having an HLB value of from 4 to 10.

2. The solid dosage form of claim 1, wherein the solid dispersion comprises a water-soluble polymer having a Tg of at least 50°C.

3. The solid dosage form of claim 1 or 2, wherein the solid dispersion is a glassy solution or solid solution.

4. The solid dosage form of claim 1, wherein the dosage form comprises a pharmaceutically acceptable surfactant besides said surfactant having an HLB value of from 4 to 10.

5. The solid dosage form of claim 4, wherein the surfactant having an HLB value of from 4 to 10 accounts for at least 50% by weight of the total amount of surfactant used.

6. The solid dosage form of claim 1, wherein said surfactant having an HLB value of from 4 to 10 is a sorbitan fatty acid ester.

7. The solid dosage form of claim 6, wherein said surfactant having an HLB value of from 4 to 10 is selected from sorbitan monolaurate and sorbitan monopalmitate.

8. The solid dosage form of claim 1, wherein the dosage form comprises, relative to the weight of the dosage form, from 5 to 30 % by weight of an HIV protease inhibitor or a combination of HIV protease inhibitors, from 50 to 85 % by weight of a water-soluble polymer or a combination of water-soluble polymers, from 2 to 20 % by weight of a surfactant or a combination of surfactants, and from 0 to 15 % by weight of additives.

9. The solid dosage form of claim 1, wherein said at least one HIV protease inhibitor is a combination of ritonavir and at least one other HIV protease inhibitor, the dosage form showing a dose-adjusted AUC of ritonavir plasma concentration in dogs of at least 9 µg.h/ml/100 mg.

10. The solid dosage form of claim 2, wherein said at least one HIV protease inhibitor is a combination of ritonavir and lopinavir.

11. The solid dosage form of claim 1, wherein the solid dispersion comprises a watersoluble polymer having a Tg of from 80 to 180 °C.

12. The solid dosage form of claim 1, wherein the dosage form comprises a homopolymer or copolymer of N-vinyl pyrrolidone.

13. The solid dosage form of claim 1, wherein the dosage form comprises a copolymer of N-vinyl pyrrolidone and vinyl acetate.

14. The solid dosage form of claim 13, wherein the dosage form comprises a copolymer of 60% by weight of the copolymer, N-vinyl pyrrolidone, and 40% by weight of the copolymer, vinyl acetate.

15. The solid dosage form of claim 1, wherein the dosage form contains at least one additive selected from flow regulators, disintegrants, bulking agents and lubricants.

16. The solid dosage form of claim 1, wherein the dosage form comprises watersoluble polymer(s) selected from homopolymers and copolymers of N-vinyl lactams, cellulose esters and cellulose ethers, high molecular polyalkylene oxides, polyacrylates and polymethacrylates, polyacrylamides, vinyl acetate polymers, partially hydrolyzed polyvinyl acetate, polyvinyl alcohol, oligo- and polysaccharides and mixtures thereof.

17. The solid dosage form of claim 1, obtainable by a method which comprises (i) preparing a homogeneous melt of said HIV protease inhibitor(s), said water-soluble polymer(s) and said surfactant(s), and (ii) allowing the melt to solidify to obtain a solid dispersion product.

18. The solid dosage form of claim 17, wherein an additive selected from flow regulators, lubricants, fillers, disintegrants, plasticizers and stabilizers is included in the melt.

19. The solid dosage form of claim 1, wherein said solid dispersion is a glassy solution or solid solution comprising ritonavir and said surfactant having an HLB value of from 4 to 10.

20. The solid dosage form of claim 1, wherein said solid dispersion is a glassy solution or solid solution comprising ritonavir, a water-soluble polymer having a Tg of at least 50°C and said surfactant having an HLB value of from 4 to 10.

21. The solid pharmaceutical dosage form of claim 1 comprising a solid dispersion of ritonavir and lopinavir;
a copolymer of N-vinyl pyrrolidone and vinyl acetate; and
a sorbitan fatty acid ester.

22. The solid dosage form of claim 21, wherein the dosage form contains at least one additive selected from flow regulators, disintegrants, bulking agents and lubricants.

23. The solid pharmaceutical dosage form of claim 1 comprising a solid dispersion of
ritonavir and lopinavir present in an amount from 5 % to 30 % by weight of the dosage form;
a copolymer of N-vinyl pyrrolidone and vinyl acetate from 50 % to 85 % by weight of the dosage form; and
a sorbitan fatty acid ester from 2 % to 20 % by weight of the dosage form.

24. The solid dosage form of any one of claims 1 to 23 for use in the treatment of an HIV infection in a mammal.

## Patentansprüche

1. Feste pharmazeutische Darreichungsform, die Folgendes umfasst: eine feste Dispersion aus mindestens einem HIV-Protease-Hemmer in mindestens einem pharmazeutisch akzeptablen wasserlöslichen Polymer und mindestens ein pharmazeutisch akzeptables Tensid, wobei die genannte Darreichungsform Ritonavir und ein Tensid umfasst, das einen HLB-Wert von 4 bis 10 aufweist.

2. Feste Darreichungsform nach Anspruch 1, wobei die feste Dispersion ein wasserlösliches Polymer umfasst, das einen Tg-Wert von mindestens 50 °C aufweist.

3. Feste Darreichungsform nach Anspruch 1 oder 2, wobei die feste Dispersion eine glasige Lösung oder eine feste Lösung ist.

4. Feste Darreichungsform nach Anspruch 1, wobei die Darreichungsform ein pharmazeutisch akzeptables Tensid umfasst und das genannte Tensid einen HLB-Wert von 4 bis 10 aufweist.

5. Feste Darreichungsform nach Anspruch 4, wobei das Tensid, das einen HLB-Wert von 4 bis 10 aufweist, mindestens 50 Gewichts-% der Gesamtmenge des verwendeten Tensids ausmacht.

6. Feste Darreichungsform nach Anspruch 1, wobei das genannte Tensid, das einen HLB-Wert von 4 bis 10 aufweist, ein Sorbitanfettsäureester ist.

7. Feste Darreichungsform nach Anspruch 6, wobei das genannte Tensid, das einen HLB-Wert von 4 bis 10 aufweist, aus Sorbitanmonolaurat und Sorbitanmonopalmitat ausgewählt wird.

8. Feste Darreichungsform nach Anspruch 1, wobei die Darreichungsform bezogen auf das Gewicht der Darreichungsform Folgendes umfasst: 5 bis 30 Gewichts-% eines HIV-Protease-Hemmers oder einer Kombination aus HIV-Protease-Hemmern, 50 bis 85 Gewichts-% eines wasserlöslichen Polymers oder einer Kombination aus wasserlöslichen Polymeren, 2 bis 20 Gewichts-% eines Tensids oder einer Kombination aus Tensiden und 0 bis 15 Gewichts- % von Additiven.

9. Feste Darreichungsform nach Anspruch 1, wobei der genannte mindestens eine HIV-Protease-Hemmer aus einer Kombination aus Ritonavir und mindestens einem anderen HIV-Protease-Hemmer besteht, wobei die Darreichungsform einen dosisangepassten AUC-Wert von Ritonavir-Plasmakonzentration bei Hunden von mindestens 9 µg.h/ml/100 mg zeigt.

10. Feste Darreichungsform nach Anspruch 2, wobei der genannte mindestens eine HIV-Protease-Hemmer aus einer Kombination aus Ritonavir und Lopinavir besteht.

11. Feste Darreichungsform nach Anspruch 1, wobei die feste Dispersion ein wasserlösliches Polymer umfasst, das einen Tg-Wert von 80 bis 180 °C aufweist.

12. Feste Darreichungsform nach Anspruch 1, wobei die Darreichungsform ein Homopolymer oder Copolymer von N-Vinylpyrrolidon umfasst.

13. Feste Darreichungsform nach Anspruch 1, wobei die Darreichungsform ein Copolymer von N-Vinylpyrrolidon und Vinylacetat umfasst.

14. Feste Darreichungsform nach Anspruch 13, wobei die Darreichungsform ein Copolymer aus 60 Gewichts-% des Copolymers, N-Vinylpyrrolidon, und 40 Gewichts-% des Copolymers, Vinylacetat, umfasst.

15. Feste Darreichungsform nach Anspruch 1, wobei die Darreichungsform mindestens ein Additiv umfasst, das aus Strömungsreglern, Desintegrationsmitteln, Füllstoffen und Gleitmitteln ausgewählt wird.

16. Feste Darreichungsform nach Anspruch 1, wobei die Darreichungsform ein oder mehrere wasserlösliche Polymere umfasst, die aus Folgendem ausgewählt sind : Homopolymeren und Copolymeren von N-Vinyllactamen, Celluloseestern und Celluloseethern, hochmolekularen Polyalkylenoxiden, Polyacrylaten und Polymethacrylaten, Polyacrylamiden, Vinylacetatpolymeren, teilweise hydrolysiertem Polyvinylacetat, Polyvinylalkohol, Oligo- und Polysacchariden und Mischungen davon.

17. Feste Darreichungsform nach Anspruch 1, die durch ein Verfahren erhältlich ist, das Folgendes umfasst: (i) Herstellen einer homogenen Schmelze des einen oder der mehreren genannten HIV-Protease-Hemmer, des einen oder der mehreren genannten wasserlöslichen Polymere und des einen oder der mehreren genannten Tenside und (ii) Verfestigen der Schmelze, um ein festes Dispersionsprodukt zu erhalten.

18. Feste Darreichungsform nach Anspruch 17, wobei ein Additiv, das aus Strömungsreglern, Gleitmitteln, Füllstoffen, Desintegrationsmitteln, Weichmachern und Stabilisatoren ausgewählt ist, in der Schmelze enthalten ist.

19. Feste Darreichungsform nach Anspruch 1, wobei die genannte feste Dispersion eine glasige Lösung oder eine feste Lösung ist, die Ritonavir und das genannte Tensid, das einen HLB-Wert von 4 bis 10 aufweist, umfasst.

20. Feste Darreichungsform nach Anspruch 1, wobei die genannte feste Dispersion eine glasige Lösung oder eine feste Lösung ist, die Folgendes umfasst: Ritonavir, ein wasserlösliches Polymer, das einen Tg-Wert von mindestens 50 °C aufweist, und das genannte Tensid, das einen HLB-Wert von 4 bis 10 aufweist.

21. Feste pharmazeutische Darreichungsform nach Anspruch 1, die eine feste Dispersion von Folgendem umfasst:
Ritonavir und Lopinavir,
ein Copolymer von N-Vinylpyrrolidon und Vinylacetat, und
ein Sorbitanfettsäureester.

22. Feste Darreichungsform nach Anspruch 21, wobei die Darreichungsform mindestens ein Additiv enthält, das aus Strömungsreglern, Desintegrationsmitteln, Füllstoffen und Gleitmitteln ausgewählt wird.

23. Feste pharmazeutische Darreichungsform nach Anspruch 1, die eine feste Dispersion von Folgendem umfasst:
Ritonavir und Lopinavir in einer Menge von 5 Gewichts-% bis 30 Gewichts-% der Darreichungsform;
ein Copolymer von N-Vinylpyrrolidon und Vinylacetat von 50 Gewichts-% bis 85 Gewichts-% der Darreichungsform; und
ein Sorbitanfettsäureester von 2 Gewichts-% bis 20 Gewichts-% der Darreichungsform.

24. Feste Darreichungsform nach einem der Ansprüche 1 bis 23 zur Verwendung bei der Behandlung einer HIV-Infektion in einem Säuger.

## Revendications

1. Forme galénique pharmaceutique solide qui comprend une dispersion solide d'au moins un inhibiteur de la protéase du VIH dans au moins un polymère soluble dans l'eau pharmaceutiquement acceptable et au moins un tensioactif pharmaceutiquement acceptable, dans laquelle ladite forme galénique comprend du ritonavir et un tensioactif ayant une valeur de HLB de 4 à 10.

2. Forme galénique solide selon la revendication 1, dans laquelle la dispersion solide comprend un polymère soluble dans l'eau ayant une Tg d'au moins 50 °C.

3. Forme galénique solide selon la revendication 1 ou la revendication 2, dans laquelle la dispersion solide est une solution vitreuse ou une solution solide.

4. Forme galénique solide selon la revendication 1, dans laquelle la forme galénique comprend un tensioactif pharmaceutiquement acceptable en plus dudit tensioactif ayant une valeur de HLB de 4 à 10.

5. Forme galénique solide selon la revendication 4, dans laquelle le tensioactif ayant une valeur de HLB de 4 à 10 représente au moins 50 % en poids de la quantité totale de tensioactif utilisée.

6. Forme galénique solide selon la revendication 1, dans laquelle ledit tensioactif ayant une valeur de HLB de 4 à 10 est un ester d'acide gras et de sorbitane.

7. Forme galénique solide selon la revendication 6, dans laquelle ledit tensioactif ayant une valeur de HLB de 4 à 10 est choisi entre le monolaurate de sorbitane et le monopalmitate de sorbitane.

8. Forme galénique solide selon la revendication 1, dans laquelle la forme galénique comprend, par rapport au poids de la forme galénique, de 5 à 30 % en poids d'un inhibiteur de la protéase du VIH ou d'une combinaison d'inhibiteurs de la protéase du VIH, de 50 à 85 % en poids d'un polymère soluble dans l'eau ou d'une combinaison de polymères solubles dans l'eau, de 2 à 20 % en poids d'un tensioactif ou d'une combinaison de tensioactifs et de 0 à 15 % en poids d'additifs.

9. Forme galénique solide selon la revendication 1, dans laquelle ledit au moins un inhibiteur de la protéase du VIH est une combinaison de ritonavir et d'au moins un autre inhibiteur de la protéase du VIH, la forme galénique révélant une ASC ajustée à la dose de la concentration du ritonavir dans le plasma de chiens d'au moins 9 µg.h/ ml/ 100 mg.

10. Forme galénique solide selon la revendication 2, dans laquelle ledit au moins un inhibiteur de la protéase du VIH est une combinaison de ritonavir et de lopinavir.

11. Forme galénique solide selon la revendication 1, dans laquelle la dispersion solide comprend un polymère soluble dans l'eau ayant une Tg de 80 à 180 °C.

12. Forme galénique solide selon la revendication 1, dans laquelle la forme galénique comprend un homopolymère ou copolymère de N-vinyl-pyrrolidone.

13. Forme galénique solide selon la revendication 1, dans laquelle la forme galénique comprend un copolymère de N-vinyl-pyrrolidone et d'acétate de vinyle.

14. Forme galénique solide selon la revendication 13, dans laquelle la forme galénique comprend un copolymère de 60 % en poids du copolymère N-vinyl-pyrrolidone et 40 % en poids du copolymère acétate de vinyle.

15. Forme galénique solide selon la revendication 1, dans laquelle la forme galénique contient au moins un additif choisi parmi des régulateurs d'écoulement, des délitants, des agents de charge et des lubrifiants.

16. Forme galénique solide selon la revendication 1, dans laquelle la forme galénique comprend un ou des polymère(s) soluble(s) dans l'eau choisis parmi des homopolymères et copolymères de lactames N-vinyliques, des esters de cellulose et des éthers de cellulose, des oxydes de polyalkylène à haute masse moléculaire, des polyacrylates et des polyméthacrylates, des polyacrylamides, des polymères d'acétate de vinyle, de l'acétate de polyvinyle partiellement hydrolysé, de l'alcool polyvinylique, des oligo- et polysaccharides et des mélanges de ceux-là.

17. Forme galénique solide selon la revendication 1, pouvant être obtenue par un procédé qui comprend les étapes consistant à (i) préparer un produit fondu homogène dudit ou desdits inhibiteur(s) de la protéase du VIH, dudit ou desdits polymère(s) soluble(s) dans l'eau et dudit ou desdits tensioactif(s) et (ii) laisser le produit fondu se solidifier pour obtenir un produit de dispersion solide.

18. Forme galénique solide selon la revendication 17, dans laquelle un additif choisi parmi des régulateurs d'écoulement, des lubrifiants, des agents de charge, des délitants, des plastifiants et des stabilisants est inclus dans le produit fondu.

19. Forme galénique solide selon la revendication 1, dans laquelle ladite dispersion solide est une solution vitreuse ou une solution solide comprenant du ritonavir et ledit tensioactif ayant une valeur de HLB de 4 à 10.

20. Forme galénique solide selon la revendication 1, dans laquelle ladite dispersion solide est une solution vitreuse ou une solution solide comprenant du ritonavir, un polymère soluble dans l'eau ayant une Tg d'au moins 50 °C et ledit tensioactif ayant une valeur de HLB de 4 à 10.

21. Forme galénique pharmaceutique solide selon la revendication 1 comprenant une dispersion solide de ritonavir et de lopinavir ;
un copolymère de N-vinyl-pyrrolidone et d'acétate de vinyle ; et
un ester d'acide gras et de sorbitane.

22. Forme galénique solide selon la revendication 21, dans laquelle la forme galénique contient au moins un additif choisi parmi des régulateurs d'écoulement, des délitants, des agents de charge et des lubrifiants.

23. Forme galénique pharmaceutique solide selon la revendication 1 comprenant une dispersion solide de
ritonavir et de lopinavir présente dans une quantité de 5 % à 30 % en poids de la forme galénique ;
un copolymère de N-vinyl-pyrrolidone et d'acétate de vinyle de 50 % à 85 % en poids de la forme galénique ; et
un ester d'acide gras et de sorbitane de 2 % à 20 % en poids de la forme galénique.

24. Forme galénique solide selon l'une quelconque des revendications 1 à 23 pour utilisation dans le traitement d'une infection par le VIH chez un mammifère.
